(19) **Europäisches Patentamt**

**European Patent Office**

**Office européen des brevets**

(11) **EP 1 500 946 A1**

(12) **EUROPEAN PATENT APPLICATION**
published in accordance with Art. 158(3) EPC

(43) Date of publication:
**26.01.2005 Bulletin 2005/04**

(51) Int Cl.⁷: **G01R 33/60**, G01N 24/10, G01N 33/02

(21) Application number: **02775476.1**

(22) Date of filing: **01.11.2002**

(86) International application number:
**PCT/JP2002/011469**

(87) International publication number:
**WO 2003/091744 (06.11.2003 Gazette 2003/45)**

(84) Designated Contracting States:
**AT BE BG CH CY CZ DE DK EE ES FI FR GB GR IE IT LI LU MC NL PT SE SK TR**

(30) Priority: **26.04.2002 JP 2002126287**

(71) Applicants:
• **Yoshikawa, Toshikazu**
  **Kyoto 611-0013 (JP)**
• **Mariyama, Toshikazu**
  **Tokyo 110-0003 (JP)**

• **Nakagami, Shizuo**
  **Warabi-shi Saitama 335-0004 (JP)**

(72) Inventor: **YOSHIKAWA, Toshikazu**
  **Uji-shi, Kyoto 611-0013 (JP)**

(74) Representative: **Dawson, Elizabeth Ann et al**
  **A.A. Thornton & Co.**
  **235 High Holborn**
  **London WC1V 7LE (GB)**

(54) **METHOD OF EVALUATING ANTIOXIDATION ABILITY BY MEASURING REDOX BALANCE IN VIVO**

(57) Provided is a method of evaluation of antioxidation level through determination of in-vivo redox balance. The redox balance is represented by a formula: redox balance = (antioxidation index after administration/oxidation index after administration)/(antioxidation index before administration/oxidation index before administration). The antioxidation index is obtained by indexing the antioxidation level that is obtained through quantitative determination of the signal intensity attenuation with serum addition thereto of the DMPO-OOL radical formed through trapping of a butylhydroperoxide radical by 5,5-dimethyl-1-pyrroline-N-oxide (DMPO) by the use of a magnetic spin resonator followed by analysis of the data for 10-grade frequency distribution of from 1 to 10, and the oxidation index is obtained by indexing the oxidation level that is obtained through quantitative determination of 8-hydroxydeoxyguanosine (8-OHdG) in urine followed by analysis of the data for 10-grade frequency distribution of from 1 to 10.

EP 1 500 946 A1

**Description**

TECHNICAL FIELD

The present invention relates to a technique for determination of the antioxidation revel of foods and/or chemicals, in particular, to a method of evaluation of the antioxidation level of foods and/or chemicals through determination of in-vivo redox balance.

BACKGROUND OF THE ART

Heretofore known are various methods for determination of the antioxidation level of foods and chemicals. For example, known are a method of quantifying active oxygen species before and after administration of foods and/or chemicals according to absorptiometry or chemiluminescence or by the use of amagnetic spin resonator to thereby determine the active oxygen removability of the intended foods and chemicals; and a method of quantifying the peroxide formed through lipid oxidation or measuring the dissolved oxygen concentration change to thereby determine the active oxygen removability of the intended foods and chemicals.

For in-vitro evaluation of the antioxidation level of foods and chemicals, detailed investigations including determination of the reaction speed constant thereof with active oxygen have already been realized. In fact, however, when foods and/or chemicals are actually administered to living bodies, it is not clear as to whether the antioxidation level measured indicates the function of the food or chemical itself or indicates the effect of the degraded or metabolized product of the food or chemical. The influence of food or chemical ingestion on biological responses must be taken into consideration, but it is difficult to take the matter into consideration in the determination according to conventional methods. Therefore, at present, when foods and/or chemicals are administered to living bodies, the matter as to whether they could actually exhibit some antioxidation capability to what degree after all remains conjectural.

With the recent development in alternative therapy, the in-vivo effectiveness of some foods and drugs in Oriental medicine such as herbal medicines for life style-related diseases such as cancer, diabetes and arteriosclerosis is suggested, but the mechanism thereof is not as yet completely clarified. Therefore needed is a method of in-vivo quantitative evaluation of foods and drugs in Oriental medicine such as herbal medicines as to whether and to what degree they exhibit their antioxidation capability in vivo.

SUMMARY OF THE INVENTION

The invention provides a method of evaluating the antioxidation level of foods and/or chemicals, which comprises administering a food and/or a chemical to animals all having the same gene and raised in the same environment, measuring the redox balance in the serum or urine of the animals before and after the food and/or chemical administration to them, and determining the antioxidation level of the food and/or the chemical based on the thus-measured redox balance. According to the method of the invention, it is possible to measure the redox balance before and after food and/or chemical administration, irrespective of food and/or chemical absorption, metabolism and biological responses, and to quantitatively evaluate the antioxidation level of foods and/or chemicals.

We, the present inventors have assiduously studied and, as a result, have found that the redox balance derived from the antioxidation index and the oxidation index before and after food and/or chemical administration, which is defined by the formula mentioned below, is extremely useful in evaluating the antioxidation level of foods and/or chemicals.

Redox Balance

= (antioxidation index after administration/oxidation index

after administration)/(antioxidation index before

administration/oxidation index before administration).

In this, the antioxidation index is obtained by indexing the radical removability of serum that is determined in accordance with a novel method by the use of a magnetic spin resonator, within a range of from 1 to 10 points, as will be described hereinunder.

The oxidation index is obtained by indexing the in-vivo oxidation degree that is obtained through quantification of 8-hydroxydeoxyguanosine (8-OHdG) in urine to determine the in-vivo oxidative DNA damage, within a range of from 1 to 10 points.

The redox balance may be a value of from 0.01 to 100. When the value is over 1.0, then it is judged that the administered food and/or chemical has an antioxidation ability. When the redox balance is 100, then it is judged that the antioxidation ability of the administered food and/or chemical is the best. In that manner, the antioxidation level of foods and/or chemicals can be quantitatively determined.

BEST MODES OF CARRYING OUT THE INVENTION

In this invention, used are animals having the same gene and raised in the same environment. To these animals, administered is a food and/or a chemical that is to be evaluated in point of its antioxidation level, and the serum and the urine collected from each animal before and after the food and/or chemical administration is analyzed to determine the redox balance thereof. Based on the thus-determined data, the antioxidation level of the food and/or the chemical tested is evaluated, and the invention provides the antioxidation level evaluation method.

The redox balance to be used in evaluating the antioxidation level is defined by the following formula:

Redox Balance

= (antioxidation index after administration/oxidation index

after administration)/(antioxidation index before

administration/oxidation index before administration).

<Antioxidation Index>

The antioxidation index is obtained by indexing the radical removability, R that is determined by the use of a magnetic spin resonator in the manner mentioned below, within a range of from 1 to 10 points. The determination with a magnetic spin resonator is effected as follows: The DMPO-OOL radical resulting from trapping of a butylhydroperoxide radical by a spin trapping agent, 5,5-dimethyl-1-pyrroline-N-oxide (DMPO) is quantitatively determined by the use of a magnetic spin resonator, and a serum sample is added to the system to determine the degree of signal intensity attenuation in the system. The radical removability, R is derived from the 50 % inhibition of signal intensity and the DMPO concentration according to the formula mentioned below. Radical Removability R = [DMPO]/50 % Inhibition Concentration.

Preferably, the determination with a magnetic spin resonator is effected as follows: 100 mM butylhydroperoxide, 50 $\mu$g/ml methemoglobin, 10 mM DMPO and 0.1 mM diethylenetriamine-pentaacetic acid are reacted in 10 mM phosphate buffer. One minute after the start of the reaction, the DMPO-OOL signal intensity is measured by the use of a magnetic spin resonator.

The radical removability R of various radical scavengers determined according to the above-mentioned method is shown below.

Table 1

| Radical Removability of Various Radical Scavengers | | |
|---|---|---|
| Scavenger | 50 % Inhibition Concentration | R |
| Cu,Zn-DOD | >100 U/ml | - |
| Catalase | >4,000 U/ml | - |
| $\beta$-Carotene | >0.2 mg/ml | - |
| Ascorbic acid | $4.4 \times 10^{-5}$ M | 227 |
| Uric acid | $3.6 \times 10^{-4}$ M | 28 |
| $\alpha$-Tocopherol | $7.0 \times 10^{-4}$ M | 14 |
| Glucose | >4.5 $\times 10^{-3}$ M | - |
| Glutathione | $4.1 \times 10^{-4}$ M | 24 |
| Albumin | >5.0 g/ml | - |

Table 1   (continued)

| Radical Removability of Various Radical Scavengers | | |
|---|---|---|
| Scavenger | 50 % Inhibition Concentration | R |
| Bilirubin | >10 mg/dl | - |

Advantageously over the prior art, the method of the invention is characterized in that the radical removability in serum and the like can be determined from the signal intensity inhibition F ($0 < F < 1$). Specifically, in the invention, the radical removability that corresponds to 1 M DMPO is defined as 1 U/L, and on the basis of it, the radical removability in serum can be quantitatively determined. The mean value of the data of 9 healthy adult panelists tested according to the method of the invention was $0.293 \pm 0.014$ U/L.

<Oxidation Index>

Abnormal gene expression is now specifically noted as a phenomenon that has a close correlation to the causes of cancer, diabetes and arteriosclerosis. Some recent studies have clarified that abnormal gene expression relating to apoptosis or cell spin is a trigger of contraction and development of diseases. The causes of such abnormal gene expression include environmental factors and oxidation stress that results from inflammation and the like, and the most typical and reliable oxidative DNA damage index for them is 8-hydroxydeoxyguanosine (8-OHdG). In the invention, the in-vivo oxidation degree that is determined through quantification of 8-hydroxydeoxyguanos ine (8-OHdG) in urine is indexed within a range of from 1 to 10 points to be an oxidation index.
The quantitative determination of 8-OHdG is effected through enzyme immunoassay using a monoclonal antibody.
Test data of diabetic animals are shown below, indicating that the determination of 8-OHdG in urine is extremely useful for evaluation of the antioxidation level of foods.

Animal Test

Leptin receptor-defective, natural diabetic db/db mice (animal model similar to human type-II diabetes) were used in the test. A group of leptin receptor-defective, natural diabetic db/db mice were fed with 0.02 % astaxanthin feed in which the in-vitro antioxidation capability of astaxanthin (a type of carotenoid dye, and this is much in marine *Crustacea*) was proved. A control group of leptin receptor-defective, natural diabetic db/db mice was not given astaxanthin. Six-week db/db mice of the two types were fed for 3 months in that manner, and the results are shown in Table 2.

Table 2

| Effect of Astaxanthin to Leptin Receptor-Defective, Natural Diabetic db/db Mice | | |
|---|---|---|
| Group | Albumin in Urine (μg/day) | 8-OHdG in Urine (ng/day) |
| Control Group | 234.4 + 71.6 | 336.1 + 68.7 |
| Astaxanthin Group | 68.6 + 13.4# | 135.6 + 41.1# |

#$p < 0.05$ vs control group

As in Table 2, the amount of albumin discharge in urine increased with time in the db/db mice of control group not given astaxanthin. Though not shown, the proliferation of mesangial cells is seen in the kidney tissue image of the db/db mice of control group, and this indicates that the pathological condition of these mice is similar to human diabetic nephropathy. From the test, it is understood that astaxanthin having the ability of antioxidation significantly inhibits 8-OHdG discharge in urine and significantly retards the development of diabetic nephropathy. From the above, it is obvious that the determination of 8-OHdG in urine may be a useful index of oxidative DNA damage in mice.
The method of the invention is readily applicable to human urine. The data of healthy adult panelists are shown below.

Table 3

| 8-OHdG Discharge in Urine of Healthy Adult Panelists | | |
|---|---|---|
| Sex | Number | 8-OHdG Concentration (µg/mg creatine) |
| Men | 25 | $6.818 \pm 0.364$ |
| Women | 34 | $8.394 \pm 0.376$ |

The data are in terms of mean value ± standard error.

As in the above, the method of determination of 8-OHdG in urine is useful as an index of oxidative DNA damage both in animals and humans, and it is obvious that the method is useful for determination of the oxidation level of foods and/or chemicals.

More generally, a lot of panelists and test animals (200 human panelists, and 100 animals) are tried for the purpose of establishing a screening method for the antioxidation level of foods and/or chemicals in the same manner as that for the determination of the antioxidation index as above, and the data are analyzed for 10-grade frequency distribution of from 1 to 10. Concretely, point 1 is given to the group having the lowest data, and point 10 to the group having the highest data; and the oxidation index is determined from the frequency distribution.

<Determination of Redox Balance and Evaluation of Antioxidation Level>

The data of antioxidation level and oxidation level obtained in the manner as above is introduced into the following formula to obtain the redox balance of the sample tested.

Redox Balance

= (antioxidation index after administration/oxidation index

after administration)/(antioxidation index before

administration/oxidation index before administration).

The redox balance may be a value of from 0.01 to 100. When the value is over 1.0, then it is judged that the administered food and/or chemical has an antioxidation ability. When the redox balance is 100, then it is judged that the antioxidation ability of the administered food and/or chemical is the best.

The method of the invention as above enables quantitative evaluation of the antioxidation level of foods and/or chemicals through determination of the redox balance before and after food administration irrespective of food and/or chemical absorption, metabolism and biological responses.

**Claims**

**1.** A method for evaluation of the antioxidation level of foods and/or chemicals, which comprises;
   a step of determining the redox balance by the use of serum and urine before and after food and/or chemical administration, and
   a step of evaluating the antioxidation level of foods and/or chemicals from the redox balance, wherein;
   the redox balance is represented by the following formula:

redox balance

= (antioxidation index after administration/oxidation index

after administration)/(antioxidation index before

administration/oxidation index before administration),

in which the antioxidation index is obtained by indexing the antioxidation level that is obtained through quantitative determination of the signal intensity attenuation with serum addition thereto of the DMPO-OOL radical formed through trapping of a butylhydroperoxide radical by 5,5-dimethyl-1-pyrroline-N-oxide (DMPO) by the use of a magnetic spin resonator followed by analysis of the data for 10-grade frequency distribution of from 1 to 10, the oxidation index is obtained by indexing the oxidation level that is obtained through quantitative determination of 8-hydroxydeoxyguanosine (8-OHdG) in urine followed by analysis of the data for 10-grade frequency distribution of from 1 to 10.

2. The method as claimed in claim 1, wherein;
the quantitative determination by the use of a magnetic spin resonator includes;
a step of reacting 100 mM butylhydroperoxide, 50 μg/ml methemoglobin, 10 mM DMPO and 0.1 mM diethylenetriamine-pentaacetic acid in 10 mM phosphate buffer,
a step of measuring the signal intensity from the DMPO-OOL radical by the use of a magnetic spin resonator, one minute after the start of the reaction, and
a step of obtaining the radical oxidation level from the following formula:

$$\text{radical oxidation level}$$

$$= \text{DMPO concentration/signal intensity 50 \% inhibition}$$

$$\text{concentration.}$$

3. The method as claimed in claim 1, wherein;
the quantitative determination of 8-hydroxydeoxyguanosine (8-OHdG) is effected through enzyme immunoassay using a monoclonal antibody.

## INTERNATIONAL SEARCH REPORT

| | International application No. |
|---|---|
| | PCT/JP02/11469 |

A. CLASSIFICATION OF SUBJECT MATTER
    Int.Cl⁷ G01R33/60, G01N24/10, G01N33/02

According to International Patent Classification (IPC) or to both national classification and IPC

B. FIELDS SEARCHED

Minimum documentation searched (classification system followed by classification symbols)
    Int.Cl⁷ G01R33/20-33/64, G01N24/00-24/14, G01N33/00-33/98

Documentation searched other than minimum documentation to the extent that such documents are included in the fields searched
    Jitsuyo Shinan Koho          1922–1996   Toroku Jitsuyo Shinan Koho   1994–2003
    Kokai Jitsuyo Shinan Koho    1971–2003   Jitsuyo Shinan Toroku Koho   1996–2003

Electronic data base consulted during the international search (name of data base and, where practicable, search terms used)
    JOIS[KOSANKANOU, SANKANOU, DENSHI SUPIN KYOMEI, DMPO, 8-OHdG, KESSEI,
    NYO](in Japanese), DIALOG[antioxidant activity, oxidant activity, ESR,
    DMPO, 8-OHdG, serum, urine]

C. DOCUMENTS CONSIDERED TO BE RELEVANT

| Category* | Citation of document, with indication, where appropriate, of the relevant passages | Relevant to claim No. |
|---|---|---|
| A | JP 4-169197 A (JEOL Ltd.),<br>17 June, 1992 (17.06.92),<br>Full text; Figs. 1 to 8<br>(Family: none) | 1,2 |
| A | JP 4-135484 A (Nikken Foods Co., Ltd.),<br>08 May, 1992 (08.05.92),<br>Full text; Figs. 1 to 4<br>(Family: none) | 1,3 |
| A | Toru Tanigawa, "Determination of hydroxyl radical scavenging activity by electron spin resonance", Journal of Kyoto Prefectural University of Medicine, 1990, Vol.99, No.1, pages 133 to 143 | 1,2 |

☒ Further documents are listed in the continuation of Box C.    ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 08 January, 2003 (08.01.03) | 28 January, 2003 (28.01.03) |

| Name and mailing address of the ISA/ | Authorized officer |
|---|---|
| Japanese Patent Office | |
| Facsimile No. | Telephone No. |

Form PCT/ISA/210 (second sheet) (July 1998)

## INTERNATIONAL SEARCH REPORT

International application No.

PCT/JP02/11469

| C (Continuation). DOCUMENTS CONSIDERED TO BE RELEVANT | | |
|---|---|---|
| Category* | Citation of document, with indication, where appropriate, of the relevant passages | Relevant to claim No. |
| A | JP 2001-200250 A (Mitsui Seito Kabushiki Kaisha), 24 July, 2001 (24.07.01), Full text; Figs. 1 to 2 (Family: none) | 1-3 |
| A | JP 2002-27929 A (Kabushiki Kaisha Genmai Koso), 29 January, 2002 (29.01.02), Full text (Family: none) | 1,2 |

Form PCT/ISA/210 (continuation of second sheet) (July 1998)